# EUROPÄISCHE PATENTANMELDUNG

(11) **EP 2 020 247 A2**
(43) Veröffentlichungstag der Anmeldung: **04.02.2009**
(21) Anmeldenummer: 08012079.3
(22) Anmeldetag: 04.07.2008
(51) Int. Cl.: A61M 1/16, A61M 1/36

(54) **Konditionierung von Blut eines Patienten durch Gase**

(30) Priorität: 31.07.2007 DE 102007038121
(71) Anmelder: NovaLung GmbH, 72379 Hechingen (DE)
(72) Erfinder: Matheis, Georg, 72393 Burladingen (DE); Maurer, Andreas, 72072 Tübingen (DE); Roberts, Gareth, Cambridge CB225EL (GB)
(74) Vertreter: Laufer, Gabriele

(57) **Zusammenfassung**

Die vorliegende Erfindung betrifft die Verwendung von Gasaustausch-Modulen zur Einstellung eines pH-Wertes eines Patienten und/oder eines isoliert perfundierten Zielgebiets/Organs. Dabei ist bevorzugt, wenn das Gasaustausch-Modul eine Begasungsmembran aufweist, insbesondere eine Hohlfaser-Membran oder eine Flachmembran. Mit dem Einsatz des Gasaustauscher-Moduls ist es bspw. möglich, einen unphysiologischen pH-Wert bei Patienten einzustellen, die mit Pharmaka behandelt werden, deren Wirkungsoptimum in einem unphysiologischen pH-Bereich liegt. Andererseits ist es durch den Einsatz der Gasaustauscher-Module möglich, Patienten zu behandeln, die bspw. eine chronische Acidose oder Alkalose besitzen, und zwar indem der pH-Wert, durch den die Acidose oder die Alkalose hervorgerufen wird, auf einen physiologischen Wert gebracht wird.

## Beschreibung

Die vorliegende Erfindung betrifft allgemein die Unterstützung regenerativer Therapien von verletzten Geweben/Organen im Körper eines Patienten sowie die Unterstützung von Pharmakotherapien oder Gentherapien bei einem Patienten. Dies umfasst Blutkreisläufe außerhalb und innerhalb des Körpers, getrennt vom Organismus perfundierte Organe und die Behandlung von entnommenem Blut.

Viele pathologische Prozesse, Verletzungen und Infektionen verursachen eine Gefährdung, eine Beschädigung oder sogar das Absterben von Geweben und/oder Organen im menschlichen Körper.

Während die unmittelbaren klinischen Gefahren, die hiervon herrühren, oftmals behandelt oder die Zustände stabilisiert werden können, bleiben längerfristige Schäden oder Beeinträchtigungen bei Patienten zurück, mit denen Sie im schlimmsten Falle ein Leben lang zurecht kommen müssen.

Medizinische regenerative Therapien basieren auf zellulären Ansätzen, bei denen Zellen oder andere Faktoren in den Patienten eingeführt werden, um Reparaturmechanismen und das Wachstum von neuen Zellen zu stimulieren, wodurch die durch den Eingriff, bzw. die Schädigung oder Beeinträchtigung hervorgerufenen Probleme der Gewebe/Organe behandelt werden sollen.

In wissenschaftlichen Studien konnte gezeigt werden, dass der Körper während des Schädigungs-Prozesses mit einer lokal begrenzten oder systemischen Entzündungsreaktion antwortet. Diese Entzündungsreaktion kann die körpereigenen Reparatursysteme stimulieren und kann auch als Stimulus für regenerative medizinische Therapien ausgenutzt werden. Es wurde jedoch auch gezeigt, dass hohe oder übermäßige Entzündungsreaktionen Gewebe oder Organe beschädigen können, und auch die Effektivität der medizinischen regenerativen Therapien negativ beeinflussen können, oder sogar zu deren Versagen führen können.

Darüber hinaus wurde zwischenzeitlich herausgefunden, dass der pH-Wert im Körper für die Funktionalität von Zellen/Geweben eine große Rolle spielt. Nicht in allen Bereichen des Körpers ist der pH-Wert gleich, sein Wert ist aber in den jeweiligen Organen/Geweben von entscheidender Bedeutung, denn nur dann können in dem jeweiligen Organ die chemischen Reaktionen unter idealen Bedingungen ablaufen. Der pH-Wert wirkt sich u.a. auf die Struktur von Zellbestandteilen, die Durchlässigkeit von Zellwänden und den Auf- und Abbau von Proteinen aus. Ferner ist er für die Wirksamkeit von Hormonen und Enzymen und die Verteilung von Elektrolyten wichtig. Besonders wichtig ist der pH-Wert für das Blut, wo es beim gesunden Menschen kaum pH-Schwankungen gibt. Der pH-Wert des Blutes eines gesunden Menschen liegt zwischen pH 7,36 und pH 7,45. Alle Stoffwechselreaktionen sind pHabhängig und können nur innerhalb dieses Bereiches optimal ablaufen.

In diesem Zusammenhang zeigten bspw. Brooks et al., "Modulation of VEGF production by pH and glucose in retinal Muller cells", Curr. Eye Res., 1998, 17:875-882, dass die Produktion des vaskulären endothelialen Wachstumsfaktors (VEGF) in den Müller'schen Zellen der Retina durch eine Erhöhung des pH-Wertes und eine Erhöhung der Glucose gesteigert werden konnte, wohingegen mit einer Absenkung des pH-Wertes und einem Absenken der Glucose eine verminderte VEGF-Produktion erreicht werden konnte. Diese Arbeitsgruppe schloss im Zusammenhang mit ihren Ergebnissen, dass bei einer bestehenden Hypoxie sowie einer Acidose und einer Hypoglycämie, wie es bei einer schweren Gewebeischämie auftritt, Gliazellen nicht mehr in der Lage sind, die VEGF-Synthese hoch zu regulieren, wohingegen eine Alkalose oder eine eine Hyperglycämie eine durch Hypoxie induzierte VEGF-Produktion verstärken kann.

Ferner wurde bei vielen Pharmakotherapien gezeigt, dass Pharmaka bei verschiedenen pH-Werten im Körper eines Patienten, bzw. in bestimmten Organen oder Geweben, unterschiedliche Wirkung zeigen. In extremen Fällen kann die Wirkung aufgrund des falschen pH-Werts vollständig verloren gehen. Dies zeigt, dass ein pharmakologischer Ansatz oftmals nur bei bestimmten physiologischen Bedingungen erfolgreich ist.

So zeigten bspw. Kinoshita et al., "Mild alkalinization and acidification differentially modify the effects of lidocaine or mexiletine on vasorelaxation mediated by ATPsensitive K+ channels", Anesthesiology, 2001; 95:200-201, dass eine Änderung des pH-Werts in der Aorta von Ratten zu unterschiedlichen Effekten des Wirkstoffes Lidocain bei der Abnahme der Gefäßspannung führt.

Achike und Dai, "Influence of pH changes on the actions of Verapamil on cardiac excitation-contraction coupling", Eur. J. Pharmacol. ,1991, 196:77-83, zeigten, dass die Wirkung von Verapamil als Calcium-Antagonist während einer Acidose oder Alkalose im Ratten-Herzzellen, die mit Adrenalin oder Kalium stimuliert waren, gesteigert wurde, woraus geschlossen wurde, dass eine Aciose oder eine Alkalose die durch Kalium stimulierten Kontraktionen des Herzens inhibieren und damit die Wirkung von Verapamil verstärken.

Daher besteht u.a. bei der regenerativen Medizin und bei der Pharmakotherapie das große Bedürfnis, die Regeneration des verletzten oder beschädigten Gewebes, bzw. die Wirkung eines Pharmakons zu unterstützen, um eine erfolgreiche Heilung der betroffenen Gewebe/Organe und eine effektive Behandlung des Patienten mit Pharmaka zu ermöglichen.

Aufgabe der vorliegenden Erfindung ist es daher, Hilfsmittel bereit zu stellen, mit denen eine regenerative Therapie im Körper eines Patienten sowie der Einsatz von Pharmaka durch eine gezielte Einstellung des pH-Wertes auf einfache Weise und ohne große Eingriffe, die den Körper des Patienten zusätzlich belasten, unterstützt und gefördert wird.

Erfindungsgemäß wird diese Aufgabe dadurch gelöst, dass ein Gasaustauscher-Modul verwendet wird, mit dem die Einstellung des pH-Wertes von Blut eines Patienten und/oder von Blut in einem isolierten Organs gesteuert wird. Dies kann in Blutkreisläufen außerhalb des Körpers oder an isoliertem Blut geschehen.

Die der Erfindung zugrunde liegende Aufgabe wird dadurch vollkommen gelöst.

Mit dem Einsatz von Gasaustauscher-Modulen kann der pH-Wert von Blut im Organ eines Patienten schnell und einfach eingestellt werden. Der Körper des Patienten wird auf diese Weise vorteilhaft nicht mit zusätzlichen, den pH-Wert beeinflussenden Substanzen belastet.

Unter "Gasaustauscher-Modul" wird vorliegend jede Vorrichtung verstanden, mit welcher dem Blut eines Patienten bei Anschluss der Vorrichtung Gase zugeführt und/oder abgeführt bzw. ausgetauscht werden können, insbesondere Sauerstoff, Kohlendioxid und Stickstoff. So fallen bspw. Oxygenatoren, die im Zusammenhang mit Herz-Lungen-Maschinen eingesetzt werden, vorliegend unter den Begriff "Gasaustauscher-Modul". Mit Oxygenatoren wird Blut mit Sauerstoff angereichert und Kohlendioxid aus dem Blut entfernt; diese kommen bspw. bei Lungenversagen zum Einsatz oder bei der Herzchirurgie.

Das Gasaustauscher-Modul wird dabei bspw. über entsprechende Zugänge, wie bspw. über eine Kanülenlegung in die gewünschten Blutgefäße, mit dem Blutsystem eines Patienten verbunden, und das Blut im Gasaustauscher-Modul begast. Das Modul wird dabei über einen Einlass mit einer Gaszufuhr verbunden, und weist einen Gasauslass auf. Durch die Begasung mit Gasen wie CO₂,Sauerstoff, Stickstoff oder deren Mischungen kann der pH-Wert des Blutes gezielt und kontrollierbar eingestellt werden, da die Menge und die Beschaffenheit des eingesetzten Gases spezifisch auf die jeweilige Verwendung bestimmt werden kann. Dabei wird ausgenutzt, dass der pH-Wert des Plasmas und der Erythrocyten, den wichtigsten Bestandteilen des Blutes, durch verschiedene Faktoren beeinflusst werden kann, wie bspw. über die Konzentration von Kohlendioxid (CO₂): Fällt die CO₂-Konzentration, so steigt der pH-Wert. Unter normalen Umständen, d.h. bei einem gesunden Menschen, wird der pH-Wert des Blutes durch ein Puffersystem konstant gehalten.

So kann bspw. bei Patienten mit chronischer Acidose oder Alkalose eine pH-Wert-Änderung hin zu physiologischen Werten erreicht werden. Die Feststellung einer Acidose oder Alkalose kann dabei bspw. über einen Sensor im Blutstrom erfolgen, der den Gasstrom und damit den physiologischen pH-Wert steuert und regelt.

Auf der anderen Seite kann bei Patienten, die eine Therapie mit Medikamenten empfangen, deren Wirkungsoptimum außerhalb des physiologischen pH-Wertes liegt, durch den Einsatz des Gasaustauscher-Moduls eine pH-Wert-Änderung hin zu unphysiologisch sauren oder basischen Werten erfolgen, bei denen die jeweilige Therapie besser anschlägt.

Die Verwendung von Gasaustauscher-Modulen bietet darüber hinaus den großen Vorteil, dass bspw. gezielt der pH-Wert einzelner Organe, die isoliert perfundiert werden, eingestellt werden kann, so dass der pH-Wert des Bluts in den nicht perfundierten Organen und Extremitäten unbeeinflusst bleibt. Dadurch kann der pH-Wert im Körper eines Patienten schonend und leicht reguliert werden, wobei die Einstellung des pH-Wertes lokal erfolgen kann.

Daher ist bei einer Ausführungsform der erfindungsgemäßen Verwendung bevorzugt, wenn das Gasaustauscher-Modul zur Einstellung des pH-Wertes von Blut eines Patienten in einem isoliert perfundierten Zielgebiet verwendet wird.

"Getrennt/isoliert perfundiert" bedeutet ein Verfahren, bei dem in einem Zielgebiet, also bspw. einem Organ, eines menschlichen oder tierischen Körpers ein künstlicher Kreislauf etabliert und aufrechterhalten wird, der von dem Blutkreislauf des Körpers, der im Folgenden auch Körperkreislauf genannt wird, isoliert ist.

Diese sogenannte isolierte Perfusion von Organen oder Körperregionen wird seit längerem angewendet, um in dem Zielgebiet hochwirksame Medikamente zu verabreichen und dabei deren Nebenwirkungen auf den Rest des Organismus zu vermeiden, bzw. Medikamente in solch hohen Konzentrationen einzusetzen, dass bei allgemeiner Anwendung auf den ganzen Körper unzumutbar starke Nebenwirkungen und Unverträglichkeiten auftreten würden.

Unter "Zielgebiet" wird im Rahmen der vorliegenden Erfindung ein von dem restlichen Körper bezüglich des Blutkreislaufes isolierbares Organ oder eine isolierbare Körperregion, wie beispielsweise Extremitäten, also Arm oder Bein, und Becken verstanden.

Die Größe des einzusetzenden Moduls hängt dabei von der jeweiligen Verwendung ab, d.h. ob das Gasaustauscher-Modul zur pH-Wert-Einstellung des gesamten Blutvolumens eines Patienten oder lediglich für getrennt perfundiertes Zielgebiet/Organ eingesetzt werden soll. So ist bspw. ein Gasaustausch-Modul bei isoliert perfundierten Organen sinnvoll, das einen Volumenstrom von Blut von 0,1-1,5 l/min ermöglicht, und mit welchem ca. 0,01 bis 1 m² Gasaustauschfläche zur Verfügung gestellt werden. Bei der pH-Wert-Einstellung des gesamten Blutvolumens eines Patienten kann bspw. ein Modul mit 0,5 1/min bis 7 1/min eingesetzt werden, mit welchem 0,1 bis 3 m² Gasaustauschfläche bereit gestellt werden.

Insbesondere ist bevorzugt, wenn das Gasaustauscher-Modul eine Begasungsmembran aufweist, vorzugsweise eine Flachmembran, eine Hohlfasermembran oder ein Mikrofluidisches System.

Bei Begasungsmembranen bzw. bei einem Einsatz von Begasungsmembranen zur gezielten pH-Wert-Einstellung von Blut ist die Gas- von der Blutseite durch eine gasdurchlässige Membran getrennt - ähnlich wie bei der menschlichen Lunge. Der Gasaustausch findet daher entlang der gasdurchlässigen Membran durch eine Partialdruckdifferenzen der eingesetzten Gase statt. Das Gas wird dem Modul über einen Gaseinlass in die Hohlfasern, bzw. auf eine Seite der Flachmembran, zugeführt; das Blut strömt außen an den Hohlfasern, bzw. auf der anderen Seite der Flachmembran vorbei, während dessen der Gasaustausch stattfindet.

Hohlfasermembranen haben den Vorteil, dass sie eine sehr viel höhere Oberfläche (je Volumeneinheit) als Flachmembranen besitzen, was es ermöglicht, Gasaustausch-Module mit Hohlfasermembranen bei gleicher Gasaustausch-Leistung kleiner zu gestalten als Gasaustausch-Module mit Flachmembranen.

Bei Hohlfasermembranen fließt das Blut außen an den Hohlfasern vorbei, während ein Spülgas (Luft, Sauerstoff, CO2 oder andere Gasgemische) die Fasern von innen durchströmt. Zwischen Blut und Gas kommt es an der Membran aufgrund eines Konzentrationsgefälles zum Austausch der Gase, wie bspw. von Sauerstoff und Kohlendioxid. Bei Flachmembranen ist das Prinzip das gleiche.

Die Begasungsmembranen können dabei ein Material aufweisen, das ausgewählt ist aus Polypropylen (PP), Polymethylpenten (PMP), Silikon, Silikon-beschichtete, oder anderen gasdurchlässigen Membranmaterialien sein. Diese Materialien werden im Stand der Technik bereits erfolgreich und erprobt im Zusammenhang mit Gasaustauscher-Modulen eingesetzt.

Die Hohlfasermembranen können dabei in dem Gasaustauscher-Modul bspw. als Bündel, gestapelte Matten oder gerollte Matten angeordnet sein, wobei der Abstand der Hohlfasern untereinander dem jeweils gewünschten Einsatz angepasst werden kann, wobei berücksichtigt wird, dass der Abstand der Fasern untereinander den Blutwiderstand und Flussraten des Systems beeinflusst. Ferner kann vorgesehen sein, dass blutseitig eine Pumpe eingesetzt wird. Der Einsatz einer Pumpe ist jedoch nicht zwingend notwendig, da bspw. bei Patienten mit guten hämodynamischen Verhältnissen eine Begasungsmembran mit niedrigem Flusswiderstand eingesetzt werden kann, und das Blut lediglich durch den ateriovenösen Druckunterschied über die Membran geführt wird und dort mit Gasen angereichert werden kann.

Dabei ist einer Ausführungsform der erfindungsgemäßen Verwendung bevorzugt, wenn das Gasaustauscher-Modul eine künstliche Lunge oder ein Oxygenator ist.

Unter "künstliche Lunge" wird vorliegend jede Vorrichtung verstanden, die die Funktion der Lunge zeitweise oder dauerhaft über nimmt.

So kann bspw. der iLA Membranventilator der Anmelderin eingesetzt werden. Der iLA Memranventilator wird normalerweise bei einem Lungenversagen zur Beatmung außerhalb der Lunge eingesetzt. Durch den iLA Membranventilator können die schädlichen Enflüsse der mechanischen Beatmung vermindert oder sogar vermieden werden, wodurch das Risiko einer Überdehnung der Lunge und die damit verbundene weitere Schädigung der Lunge und weiterer Organe vermieden wird.

Über den iLA Membranventilator kann nun dem Blut ein Gas, wie bspw. CO₂, Sauerstoff, oder Stickstoff zugeführt werden, wobei an der in dem iLA vorgesehenen Membran der Gasaustausch stattfindet, und das Blut mit dem entsprechend zugeführten Gas angereichert wird.

Unter "Oxygenator" wird vorliegend jede medizinische Vorrichtung verstanden, mit der Sauerstoff und Kohlendioxid im Blut eines Patienten bei chirurgischen Eingriffen ausgetauscht werden kann, bei welchen der Blutstrom im Körper aus operativen Gründen unterbrochen oder gestoppt werden muss. Der Oxygenator kann dabei bspw. im Zusammenhang mit Herz-Lungen-Maschinen eingesetzt werden, oder aber bei der extrakorporalen Oxygenierung von Blut.

Beispielhafte Oxygenatoren, die für die erfindungsgemäße Verwendung eingesetzt werden können, sind Oxygenatoren der Firmen Medtronic Inc. USA, Maquet Cardiopulmonary, Deutschland, Cobe CV, USA, Sorin Biomedica, Italien. Ein Oxygenator führt dem Blut lebenswichtigen Sauerstoff zu und entfernt das durch Stoffwechselprozesse entstehende Kohlendioxid. Die Oxygenatoren weisen meist Hohlfasern auf, an denen das Blut außen vorbeiströmt, während Sauerstoff, Luft oder andere Gase die Fasern von innen durchströmen. Aufgrund eines Konzentrationsgefälles kommt es zwischen Gas und Blut an der Membran zum Austausch von Gasen, insbesondere von Sauerstoff und Kohlendioxid. Zur Aufrechterhaltung des Organismus wird das Blut wird mit Sauerstoff angereichert und vom Kohlendioxid befreit. Auf diesen Weise kann der pH-Wert des Blutes durch eine Änderung der O₂-Zufuhr oder durch einen Einsatz einer CO₂-Zufuhr über den Oxygenator gezielt eingestellt werden.

Bei einer Ausführungsform der erfindungsgemäßen Vorrichtung ist bevorzugt, wenn das Gasaustauscher-Modul zur Einstellung eines physiologischen pH-Wertes des Blutes eingesetzt wird.

Diese Ausführungsform der erfindungsgemäßen Verwendung wird vorteilhafterweise, wie bereits weiter oben erwähnt, bspw. bei Patienten eingesetzt, die unter einer Acidose oder Alkalose leiden, seien es chronische oder akute Acidosen/Alkalosen. Bei diesen Patienten ist der pH-Wert erhöht (Alkalose) bzw. erniedrigt (Acidose), wodurch Zell- und damit auch Organ- bzw. Gewebefunktionen beeinträchtigt werden können. Durch den Einsatz des Gasaustauscher-Moduls kann dem Blut der Patienten gezielt Gas zugeführt werden, wodurch der pH-Wert des Blutes wieder auf physiologische Werte eingestellt wird, d.h. auf Werte, bei denen die Zellen/Organe/Gewebe wie bei einem gesunden Menschen arbeiten. Wie weiter oben erwähnt, liegt der pH-Wert von arteriellen Blut eines gesunden Menschen im allgemeinen zwischen 7,36 und 7,45. Eine Acidose kann verschiedene Ursachen haben, wie bspw. Störungen des Gasaustausches bei Lungenerkrankungen, Erkrankungen des Gehirns, oder aber durch Stoffwechselstörungen bedingt sein, wie bspw. Niereninsuffizienz, Verbrennungen, Schock, angeborene Krankheiten. Ein erhöhter pH-Wert kann bspw. bei einem gestörten Hormonhaushalt auftreten.

Auf der anderen Seite ist in einer anderen Ausführungsform bevorzugt, wenn das Gasaustauscher-Modul zur Einstellung eines unphysiologischen pH-Wertes des Blutes eingesetzt wird.

Diese Ausführungsform hat den Vorteil, dass bspw. bei Patienten, die mit Pharmaka behandelt werden, deren Wirkungsoptimum in einem unphysiologischen pH-Wert liegt, der pH-Wert, je nach Wirkungsoptimum des eingesetzten Pharmakons, gezielt und kontrollierbar in den sauren oder basischen Bereich geführt werden kann.

Insbesondere ist bevorzugt, wenn der das Gasaustauscher-Modul zur Einstellung eines pH-Wertes des Blutes zwischen 2,5 und 9 eingesetzt wird.

Das Einzusetzende Gas, bzw. die Beschaffenheit des Gases, kann auf den jeweiligen Einsatz bzw. auf die jeweilige Verwendung abgestimmt werden. Insbesondere ist bevorzugt, wenn mit dem Gasaustauscher-Modul eine CO₂-, O₂- und/oder N₂-Zufuhr zum Blut verwendet wird.

Das Kohlendioxid ist im Blut physikalisch als Kohlensäure (HCO₃⁻) gelöst, wobei diese im Dissoziationsgleichgewicht mit CO₂ steht. Über dieses Dissoziationsgleichgewicht wird der pH-Wert im Blut geändert und bspw. bei einer vermehrten CO₂-Zufuhr vermindert, wodurch optimale Bedingungen für den jeweils durch eine pH-Wert-Einstellung zu behandelnden Patienten bzw. Organ geschaffen werden können. Durch eine Zufuhr von CO₂ kann bspw. der pH-Wert gezielt abgesenkt werden, wenn zuvor der pH-Wert des Blutes physiologisch war. Wenn der pH-Wert des zu behandelnden Blutes vor der Behandlung eher unphysiologisch basisch ist, kann der pH-Wert mit einer CO₂-Zufuhr auf einen physiologischen Wert abgesenkt werden. Umgekehrt kann bspw. mit einer O₂-Zufuhr der pH-Wert bspw. von einem unphysiologischen sauren pH-Wert angebogen werden. Auch mit einer N₂-Zufuhr kann keine Anhebung des pH-Wertes erreicht werden, so dass ein unphysiologisch saurer pH-Wert bspw. auf einen physiologischen pH-Wert angehoben werden kann, oder ein physiologischer pH-Wert auf einen unphysiologischen basischen pH-Wert.

Die Erfindung betrifft daher auch ein Verfahren zur Einstellung eines pH-Wertes von Blut in einem Patienten und/oder von Blut in einem isolierten Organ, wobei das Verfahren den Schritt des Einsatzes eines Gasaustauscher-Moduls umfasst. Dabei ist insbesondere bevorzugt, wenn das Organ in einem Patienten isoliert perfundiert ist, und wenn das Gasaustauscher-Modul zumindest eine Begasungsmembran, insbesondere eine Hohlfasermembran oder eine Flachmembran, aufweist.

Bei dem Verfahren ist ferner bevorzugt, wenn das Gasaustauscher-Modul eine künstliche Lunge oder ein Oxygenator ist.

Das Gasaustauscher-Modul wird in dem erfindungsgemäßen Verfahren zur Einstellung eines physiologischen pH-Wertes des Blutes eingesetzt wird, und kommt insbesondere dann zum Einsatz, wenn Patienten behandelt werden sollen, die unter chronischer oder akuter Acidose oder Alkalose leiden. Auf der anderen Seite kann das Verfahren auch zur Einstellung eines unphysiologischen pH-Wertes des Blutes eingesetzt werden, wenn etwa Patienten mit Pharmaka behandelt werden, deren Wirkungsoptimum im sauren oder im basischen Bereich liegt. Ferner kann das erfindungsgemäße Verfahren bzw. die erfindungsgemäße Verwendung dann zum Einsatz kommen, wenn bei Patienten, die eine Pharmakontherapie bei einem physiologsichen pH-Wert erhalten, der pH-Wert angehoben oder gesenkt werden soll, um dadurch das Pharmakon zu inaktivieren, zu eliminieren oder freizusetzen.

Mit der neuen Verwendung der Gasaustauscher-Module wird ein einfaches und effizientes Mittel bereitgestellt, mit welchem der pH-Wert des Blutes eines Patienten und/oder eines isoliert perfundierten Zielgebiets/Organs schnell, gezielt und ohne Belastung des Körpers mit zusätzlichen Substanzen eingestellt werden kann.

Weitere Vorteile ergeben sich aus der Beschreibung und den Beispielen.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Ein Ausführungsbeispiel der Erfindung wird in der nachfolgenden Beschreibung unter Bezug auf die beigefügte Zeichnung näher erläutert. Es zeigen:
- Fig. 1: die diagrammatische Darstellung der Abhängigkeit des pH-Wertes vom CO₂-Partialdruck; und
- Fig. 2: ein Diagramm, in dem die Ergebnisse eines Versuches über die Korrelation zwischen CO₂-Zufuhr und pH-Wert dargestellt sind.

### Beispiel

### Konditionierung von 200 ml Blut auf pH 7.3.

Für diesen Versuch wurde folgender Versuchsaufbau verwendet: Als Gasaustauscher-Modul wurde ein Hohlfasermodul der Anmelderin eingesetzt (Ref.: HF-PMP-90/200 Lot 2006_006). Dabei handelte es sich um ein Modul aus ca. 25 Polymethylpenten-Hohlfasern, das eine Länge von 10 cm aufwies.

Das Modul wurde mit einer Gaszufuhr und mit einem Gasauslass versehen und in ein Becherglas gegeben. Verwendet wurde nun 200 ml Blut, das vor der Behandlung einen pH-Wert von 7,35, einen Sauerstoff Partialdruck pO₂ von 40,8 und einen Kohlendioxid Partialdruck CO₂ von 48,6 aufwies. Das Blut wurde in das Becherglas gegeben, in das das Hohlfaser-Modul eingebracht war, und bedeckte dieses vollständig.

Anschließend wurde in das Hohlfaser-Modul CO₂ zugeführt, und zwar mit 0,1 L/min, um das Blut auf einen pH-Wert von <7 einzustellen. Anschließend wurde die CO₂-Zufuhr abgestellt, und eine O₂-Zufuhr von 0,2 L/min bzw. 0,5 L/min eingestellt, zur Erreichung einer maximalen O₂-Sättigung. Anschließend wurde die O₂-Begasung abgestellt.

Um den pH-Wert des Blutes auf pH >7 einzustellen und um eine minimale CO₂ / O₂ Sättigung zu erreichen, wurde eine N₂-Begasung auf 0,5 L/min eingestellt.

Alle 10 min wurden Proben entnommen und die Proben sofort einer Gasanalytik unterzerzogen.

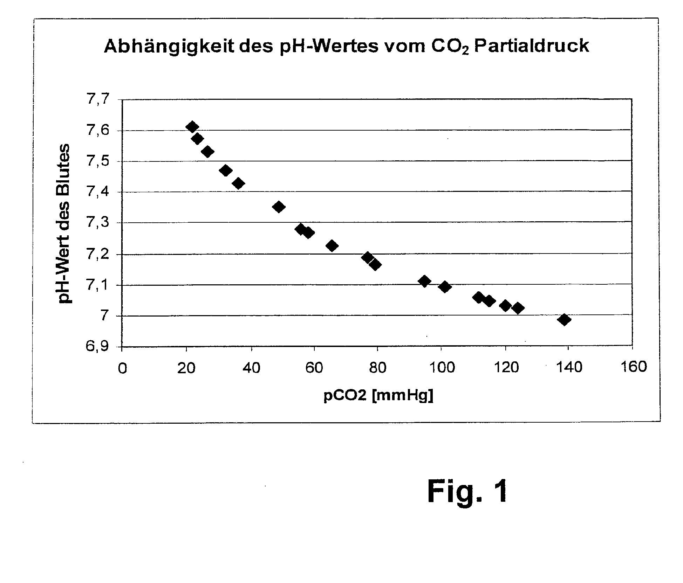

In Fig. 1 ist dargestellt, wie der pH-Wert des Blutes vom CO₂-Partialdruck p CO₂ abhängt. Zu erkennen ist, dass, je höher der Partialdruck des CO₂, desto höher der pH-Wert des Blutes ist, so dass der pH-Wert entsprechend eingestellt werden kann. Bei einem pCO₂ von 20 mm Hg beträgt der pH-Wert ca. 7,6, bei einem pCO₂ von 140 mm Hg liegt der pH-Wert bei ca. 7.

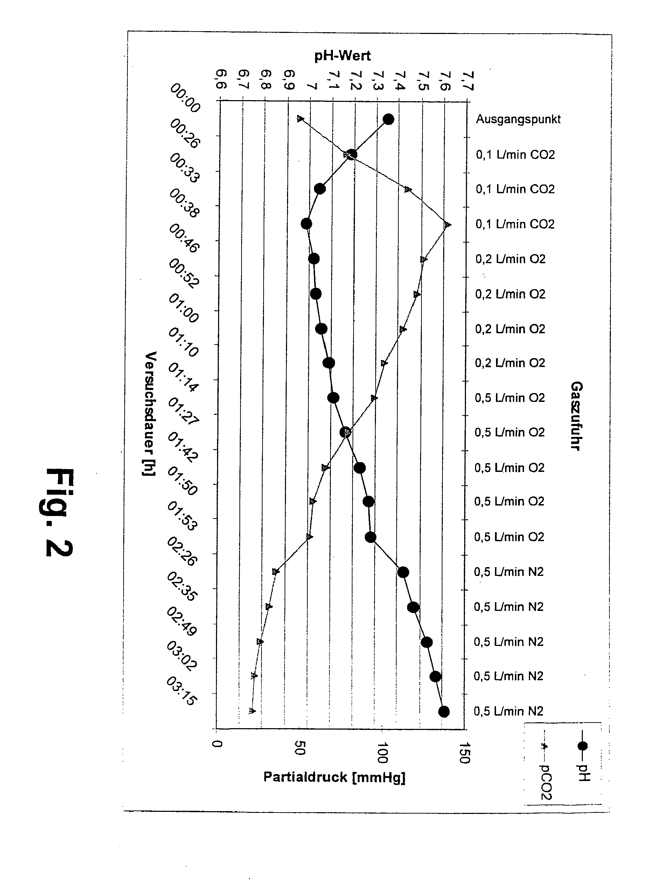

Die Ergebnisse des oben geschilderten Versuches sind auch in dem Diagramm in Fig. 2 gezeigt, in dem die beiden Kurven einmal den Verlauf des pH-Werts (ausgefüllte schwarze Kreise) zeigen und einmal den Verlauf des Partialdrucks pCO₂ (ausgefüllte helle Dreiecke). Der pH-Wert wurde gegen die Versuchsdauer und die Gaszufuhr der drei Gase aufgetragen.

Wie Fig. 1 zu entnehmen ist, konnte durch eine Zufuhr von CO₂ über das Hohlfaser-Modul zu dem Blut ein Absenken des pH-Wertes unter 7 erreicht werden (siehe die Messpunkte nach 26 min, 33 min, 38 min). Durch eine Zufuhr von O₂ wiederum konnte der pH-Wert angehoben werden, und zwar um so schneller, je großvolumiger die O₂-Zufuhr war (siehe die Messpunkte nach 46 min, 52 min, 1 h, 1 h 10 min, 1 h 14 min, 1 h 27 min, 1 h 42 min, 1 h 50 min, 1 h 53 min), und zwar bis zur maximalen CO₂ / O₂ Sättigung des Blutes. Durch die anschließende Zufuhr von N₂ konnte der pH-Wert dann auf über 7,6 gesteigert werden (siehe die Messpunkte nach 2 h 26 min, 2 h 35 min, 2 h 49 min, 3 h 02 min und 3 h 15 min).

Diese Ergebnisse zeigen, dass es durch den Einsatz von Gasaustauscher-Modulen möglich ist, den pH-Wert von Blut in einem Patienten, und auch den pH-Wert des Blutes in einem isoliert perfundierten Zielgebiet/Organ, durch die Zufuhr von Gasen gezielt zu beeinflussen, und zwar in Abhängigkeit des verwendeten Gases. Der pH-Wert kann dabei bspw. über die Volumenmenge des Gases genau vorherberechnet werden.

## Patentansprüche

1. Gasaustauschermodul zur Verwendung als Arzneimittel zur Einstellung des pH-Wertes von Blut eines Patienten und/oder von Blut in einem isolierten Zielgebiet in einem Patienten.

2. Gasaustauscher-Modul nach Anspruch 1, **dadurch gekennzeichnet, dass** das Zielgebiet ein isoliert perfundiertes Organ im Patienten ist.

3. Gasaustauscher-Modul nach Anspruch 1 oder 2, **dadurch gekennzeichnet, dass** das Gasaustauscher-Modul zumindest eine Begasungsmembran aufweist, und insbesondere eine Flachmembran oder eine Hohlfasermembran oder Mikrostrukturen ist.

4. Gasaustauscher-Modul nach einem der Ansprüche 1 bis 3, **dadurch gekennzeichnet, dass** das Gasaustauscher-Modul eine künstliche Lunge oder ein Oxygenator ist.

5. Gasaustauscher-Modul nach einem der Ansprüche 1 bis 4, **dadurch gekennzeichnet, dass** das Gasaustauscher-Modul zur Einstellung eines physiologischen oder eines unphysiologischen pH-Wertes des Blutes eingesetzt wird.

6. Gasaustauscher-Modul nach einem der Ansprüche 1 bis 5, **dadurch gekennzeichnet, dass** das Gasaustauscher-Modul zur Einstellung eines pH-Wertes des Blutes zwischen 2,5 und 9 eingesetzt wird.

7. Gasaustauscher-Modul nach einem der Ansprüche 1 bis 6, **dadurch gekennzeichnet, dass** mit dem Gasaustauscher-Modul eine CO₂-, O₂- und/oder N₂-Zufuhr zum Blut verwendet wird.

8. Verwendung eines Gasaustauscher-Moduls zur Einstellung des pH-Wertes von Blut eines Patienten und/oder von Blut in einem isolierten Zielgebiet in einem Patienten.

9. Verwendung nach Anspruch 8, **dadurch gekennzeichnet, dass** das Zielgebiet ein isoliert perfundiertes Organ im Patienten ist.

10. Verwendung nach Anspruch 8 oder 9, **dadurch gekennzeichnet, dass** das Gasaustauscher-Modul zumindest eine Begasungsmembran aufweist, und insbesondere eine Flachmembran oder eine Hohlfasermembran oder Mikrostrukturen ist.

11. Verwendung nach einem der Ansprüche 8 bis 10, **dadurch gekennzeichnet, dass** das Gasaustauscher-Modul eine künstliche Lunge oder ein Oxygenator ist.

12. Verwendung nach einem der Ansprüche 8 bis 11, **dadurch gekennzeichnet, dass** das Gasaustauscher-Modul zur Einstellung eines physiologischen oder eines unphysiologischen pH-Wertes des Blutes eingesetzt wird.

13. Verwendung nach einem der Ansprüche 8 bis 12, **dadurch gekennzeichnet, dass** das Gasaustauscher-Modul zur Einstellung eines pH-Wertes des Blutes zwischen 2,5 und 9 eingesetzt wird.

14. Verwendung nach einem der Ansprüche 8 bis 13, **dadurch gekennzeichnet, dass** mit dem Gasaustauscher-Modul eine CO₂-, O₂- und/oder N₂-Zufuhr zum Blut verwendet wird.
